# EUROPEAN PATENT APPLICATION

(11) **EP 2 286 836 A1**
(43) Date of publication of application: **23.02.2011**
(21) Application number: 09733522.8
(22) Date of filing: 17.04.2009
(51) Int. Cl.: A61K 45/00, A23L 1/30, A23L 2/52, A61K 31/132, A61K 31/135, A61K 38/00, A61K 38/16, A61P 1/04, A61P 1/12, A61P 31/00, A61P 35/00, A61P 37/04, A61P 37/08

(54) **IMMUNOSTIMULATING AGENT**

(30) Priority: 17.04.2008 JP 2008108063
(71) Applicant: Ajinomoto Co., Inc., Tokyo, 104-8315 (JP)
(72) Inventor: KODERA, Tomohiro, Kawasaki-shi Kanagawa 210-8681 (JP); ETO, Yuzuru, Kawasaki-shi Kanagawa 210-8681 (JP); MINE, Yoshinori, Shizuoka-shi Shizuoka 421-0101 (JP)
(74) Representative: Strehl Schübel-Hopf & Partner
(86) International application number: PCT/JP2009/057721
(87) International publication number: WO 2009/128523

(57) **Abstract**

Disclosed is an immunostimulating agent which can stimulate immunity effectively. The immunostimulating agent comprises, as an active ingredient, a calcium receptor activator such as γ-Glu-X-Gly [wherein X represents an amino acid or a derivative thereof other than Cys], γ-Glu-Val-Y [wherein Y represents an amino acid or a derivative thereof], γ-Glu-Ala, γ-Glu-Gly, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-y-Glu-Val, γ-Glu-Val-NH2, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, γ-Glu-Cys(S-Me), a cation having a valency of 2 or more, protamine, polylysine, spermine, spermidine, putrescine, cinacalcet, a cinacalcet analogue compound, and a salt of any one of the aforementioned components.

## Description

### Technical Field

The present invention relates to an immunostimulating agent and can be utilized in the fields of pharmaceuticals, foods, and the like.

### Background Art

Compounds such as glutathione, cinacalcet, and γ-glutamylvaline as well as derivatives thereof or analogs thereof have an action of activating a calcium sensing receptor (CaSR) and it has been implied that these CaSR agonists can be used as therapeutic agents for internal diseases (Patent Document 1).

Also, an influenza virus infection·prophylactic composition containing glutathione has been known (Patent Document 2).

However, it has not been known that the CaSR agonist can significantly stimulate immunity.
Patent Document 1: WO2007/055388
Patent Document 2: WO98/30228

### Summary of the Invention

An object of the present invention is to provide an immunostimulating agent capable of effectively stimulating immunity as well as to provide foods and beverages containing said agent.

To solve the above-mentioned problem, the present inventors intensively studied to find that compounds such as cinacalcet and γ-glutamylvaline, which are calcium receptor activators, have an immunostimulating action, thereby completing the present invention.

That is, the present invention is as follows.
(1) An immunostimulating agent containing a calcium receptor activator.
(2) The above-mentioned immunostimulating agent, wherein the calcium receptor activator is selected from the group consisting of γ-Glu-X-Gly (X represents an amino acid or an amino acid derivative other than Cys), γ-Glu-Val-Y (Y represents an amino acid or an amino acid derivative), γ-Glu-Ala, γ-Glu-Gly, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-γ-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, γ-Glu-Cys(S-Me), a cation having a valence of two or more, protamine, polylysine, spermine, spermidine, putrescine, cinacalcet, cinacalcet analogous compounds, and salts thereof.
(3) The above-mentioned immunostimulating agent, wherein the X is Cys(SNO), Cys(S-allyl), Gly, Cys(S-Me), Abu, or Ser; and the Y is Gly, Val, Glu, Lys, Phe, Ser, Pro, Arg, Asp, Met, Thr, His, Orn, Asn, Cys, or Gln.
(4) The above-mentioned immunostimulating agent, wherein the above-mentioned calcium receptor activator is γ-Glu-Val or cinacalcet.
(5) The above-mentioned immunostimulating agent, which is used as a pharmaceutical for the treatment or prophylaxis of an infection, diarrhea, polyp, tumor, enteritis, or allergy.
(6) A food or beverage for immunostimulation containing γ-Glu-Val or cinacalcet in an amount of 0.000000001 % by mass or more.
(7) A use of a calcium receptor activator for manufacturing an immunostimulating agent.
(8) The above-mentioned use, wherein the calcium receptor activator is selected from the group consisting of γ-Glu-X-Gly (X represents an amino acid or an amino acid derivative other than Cys), γ-Glu-Val-Y (Y represents an amino acid or an amino acid derivative), γ-Glu-Ala, γ-Glu-Gly, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-y-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, γ-Glu-Cys(S-Me), a cation having a valence of two or more, protamine, polylysine, spermine, spermidine, putrescine, cinacalcet, cinacalcet analogous compounds, and salts thereof.
(9) The above-mentioned use, wherein the X is Cys(SNO), Cys(S-allyl), Gly, Cys(S-Me), Abu, or Ser; and the Y is Gly, Val, Glu, Lys, Phe, Ser, Pro, Arg, Asp, Met, Thr, His, Orn, Asn, Cys, or Gln.
(10) The above-mentioned use, wherein the above-mentioned calcium receptor activator is γ-Glu-Val or cinacalcet.
(11) The above-mentioned use of the calcium receptor activator for manufacturing a pharmaceutical for the treatment or prophylaxis of an infection, diarrhea, polyp, tumor, enteritis, or allergy.
(12) A method for stimulating immunity comprising administrating a calcium receptor activator to a subject in need of stimulation of immunity.
(13) The above-mentioned method for stimulating immunity, wherein the calcium receptor activator is selected from the group consisting of γ-Glu-X-Gly (X represents an amino acid or an amino acid derivative other than Cys), γ-Glu-Val-Y (Y represents an amino acid or an amino acid derivative), γ-Glu-Ala, γ-Glu-Gly, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-γ-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, γ-Glu-Cys(S-Me), a cation having a valence of two or more, protamine, polylysine, spermine, spermidine, putrescine, cinacalcet, cinacalcet analogous compounds, and salts thereof.
(14) The above-mentioned method for stimulating immunity, wherein the X is Cys(SNO), Cys(S-allyl), Gly, Cys(S-Me), Abu, or Ser; and the Y is Gly, Val, Glu, Lys, Phe, Ser, Pro, Arg, Asp, Met, Thr, His, Orn, Asn, Cys, or Gln.
(15) The above-mentioned method for stimulating immunity, wherein the above-mentioned calcium receptor activator is γ-Glu-Val or cinacalcet.
(16) A method for treating or preventing an infection, diarrhea, polyp, tumor, enteritis or allergy, the method comprising administrating the above-mentioned calcium receptor activator to a patient suffering from infection, diarrhea, polyp, tumor, enteritis, or allergy.

### Description of the Preferred Embodiments

The present invention will now be described in detail.
An immunostimulating agent of the present invention contains a calcium receptor activator as an active ingredient.

It is preferred that the calcium receptor activator be a peptide or low molecular weight compound having an action of activating a calcium receptor.

### <1> Calcium Receptor Activator

The term "calcium receptor" as used herein refers to a receptor that is called Calcium Sensing Receptor (CaSR) and belongs to the class C of seven-transmembrane receptors. The term "calcium receptor activator" (hereinafter, also referred to as "CaSR activator") as used herein includes a substance which regulates the functions of CaSR-expressing cells by binding to the above-described CaSR and activating the CaSR (hereinafter, also referred to as "CaSR agonist") and a substance which functions to extend the CaSR activity by binding to the above-described CaSR and activating the CaSR (hereinafter, also referred to as "CaSR modulator"). Also, the term "CaSR activation" as used herein means that a ligand binds to a calcium receptor to activate a guanine nucleotide binding protein, and thereby to transduce a signal. In addition, transduction of this signal by the calcium receptor is referred to as "CaSR activity".

A CaSR activator can be obtained, for example, by a screening method shown below. Yet, the method is not limited thereto. And, the CaSR activator and screening method thereof are also described in WO2007/055388 in detail.

A CaSR agonist and modulator can be screened by examining the presence or absence of CaSR activation by a test substance using CaSR-expressing cells.
The presence or absence of CaSR activation can be examined by measuring the amount of a substance (ligand) that binds to CaSR, a substance that inhibits a reaction with a signal for regulating the CaSR activity, a substance (such as a second messenger) that transduces a signal generated by the binding of a ligand to CaSR, or the like. For instance, by detecting a second messenger generated by binding of a ligand such as Ca²⁺ to CaSR, the activation of CaSR can be detected. Also, by using a labeled known ligand, and measuring the binding between the labeled ligand and CaSR, the activation of CaSR can be detected.

CaSR to which a ligand has been bound acts on a GTP binding protein (also referred to as a G protein; Gi, Gq or the like) to control various functions of cells via second messengers such as cAMP. Among these, by the activation of Gq, the intracellular calcium concentration increases. Also, in the downstream of the increase in intracellular calcium concentration in signal transduction, functions are acutely regulated through the activation of intracellular enzymes such as calmodulin, protein kinase C, and adenylate cyclase, or through the phosphorylation of cytoplasmic proteins/membrane proteins. Thus, by bringing a test substance into contact with CaSR-expressing cells , and observing G protein activation using a measured value of the intracellular calcium concentration, the accumulated amount of intracellular cAMP, or the like as an indicator, the presence or absence of the CaSR activation by the test substance can be detected.

CaSR-expressing cells to be used for screening can be, for example, cells derived from animals including mammals such as mice, rats, hamsters, guinea pigs, rabbits, dogs, monkeys, and human beings, avian species such as chickens, and the like. Also, with regard to CaSR, the origin thereof is not particularly limited and examples thereof include CaSR of the above-mentioned animals. Specifically, a preferred example includes the human CaSR encoded by the human CaSR gene registered under GenBank Accession No. M_000388. It should be noted that CaSR is not limited to the protein encoded by the gene having the above-mentioned sequence; and can be a protein encoded by a gene having a homology of 60% or more, preferably 80% or more, more preferably 90% or more, and particularly preferably 98% or more to the above-mentioned sequence as long as the gene encodes a protein having the CaSR function. The GPRC6A receptor and the 5.24 receptor are also known as a subtype of CaSR and can be used in the method below.

A test substance in the screening can be any of known compounds and novel compounds. Examples thereof include nucleic acids, saccharides, lipids, proteins, peptides, organic low molecular weight compounds, compound libraries prepared by using combinatorial chemistry techniques, random peptide libraries prepared by solid phase synthesis or a phage display method, natural ingredients derived from microorganisms, animals and plants, marine organisms, or the like, and the like.

A first screening method (hereinafter, also referred to as "method A") comprises, for example, the following steps (a), (b), and (c):
(a) a step of bringing a test substance into contact with CaSR-expressing cells;
(b) a step of measuring the G protein activation in the cells brought into contact with the test substance and comparing this activation with the activation in control cells not brought into contact with the test substance; and
(c) a step of selecting a substance capable of activating CaSR based on the comparison results in the above-mentioned (b).

In the step (a) of the above-described screening method, the CaSR-expressing cells are kept under contact condition with the test substance. The contact of the test substance to the cells can be performed in a culture medium. The culture medium is appropriately selected depending on the kind of cells to be used and the like.

In the step (b), firstly, the activation of G proteins in the CaSR-expressing cells in the presence of the test substance is evaluated. Next, this activation is compared with activation in the absence of the test substance. Here, examples of an indicator for measuring the activation of G proteins include the intracellular calcium concentration, the amount of intracellular cAMP, and the like.

In the step (c), the comparison of the activations is conducted based on, for example, the presence or absence of a significant difference. As a result of the evaluation, if it can be confirmed that the activation is increased or extended in the presence of the test substance as compared to that in the absence of the test substance, the test substance can be assessed as a CaSR agonist.

Also, in the case of screening a CaSR modulator, in the above-mentioned step (a), the test substance and a CaSR agonist were brought into contact with the CaSR-expressing cells; in the step (b), the activation of G proteins in cases where the CaSR agonist was brought into contact with the cells in the presence of the test substance is compared with the activation of G proteins in cases where the CaSR agonist was brought into contact with the cells in the absence of the test substance; and in the step (c), a substance which extends the activation of G proteins can be selected as a CaSR modulator.

A second screening method of a CaSR agonist or modulator comprises, for example, the following steps (a), (b), and (c):
(a) a step of bringing a test substance and a ligand acting on CaSR into contact with CaSR-expressing cells;
(b) a step of measuring the amount of the ligand bound to the cell membrane of the cells and comparing this amount of the ligand with the amount of the ligand in control cells not brought into contact with the test substance; and
(c) a step of selecting a substance capable of activating CaSR based on the comparison results in the above-mentioned (b).

In the step (a) of the above-mentioned screening method, the CaSR-expressing cells are kept under contact condition with the test substance and the ligand acting on CaSR. The contact of the test substance and the ligand acting on CaSR to the cells can be performed in a culture medium. The culture medium is appropriately selected depending on the kind of cells to be used and the like.

In the step (b), firstly, the amount of the ligand bound to the cell membrane of the CaSR-expressing cells in the presence of the test substance is evaluated. Next, this amount of the ligand is compared with the amount of the ligand in the absence of the test substance. The amount of the ligand bound can be measured, for example, by using a radio-labeled ligand or the like.

In the step (c), the comparison of the amounts of the ligand is conducted based on, for example, the presence or absence of a significant difference. As a result of the evaluation, if it can be confirmed that the amount of the ligand bound is decreased in the presence of the test substance as compared to that in the absence of the test substance, the test substance can be assessed as a CaSR agonist or a CaSR modulator.

Further, as for the substance which has been confirmed to decrease the amount of the ligand bound, by the above-described screening method A, it can be confirmed that the substance is a CaSR agonist.

The ligand acting on CaSR is not particularly limited and examples thereof include Ca²⁺, cinacalcet, and the like.

Hereinafter, specific methods (1) to (3) for detecting an immunostimulating substance using CaSR-expressing cells (cells having CaSR in the state where the CaSR can function) will be exemplified.

(1) A method comprising the following steps (a), (b), and (c):
   (a) a step of bringing a test substance into contact with CaSR-expressing cells for a certain period of time;
   (b) a step of measuring the amount of cAMP in the cells brought into contact with the test substance and comparing this amount of cAMP with the amount of cAMP in control cells not brought into contact with the test substance; and
   (c) a step of selecting a substance capable of activating CaSR based on the comparison results of the above-mentioned (b).

The amount of cAMP can be measured using a commercially available assay kit.
In the step (a) of the above-mentioned screening method, in the case of screening a CaSR modulator, the test substance and a CaSR agonist can be brought into contact with the CaSR-expressing cells.
In the step (b) of the above-mentioned screening method, in the case of screening a CaSR modulator, the amount of cAMP in the case of bringing a CaSR agonist into contact with cells in the presence of the test substance can be compared with the amount of cAMP in the case of bringing the CaSR agonist into contact with the cells in the absence of the test substance.

In the step (c) of the above-mentioned screening method, the comparison of the cAMP amounts is conducted based on, for example, the presence or absence of a significant difference. As a result of the evaluation of the cAMP amount, if it can be confirmed that the cAMP amount increases, the test substance can be assessed as a substance capable of activating CaSR. In the case of screening a CaSR modulator, a substance which extends the increase in the cAMP amount can be selected as a CaSR modulator.

(2) A method comprising the following steps (a), (b), and (c):
   (a) a step of bringing a test substance and a known ligand acting on CaSR (such as Ca²⁺ or cinacalcet) into contact with CaSR-expressing cells for a certain period of time;
   (b) a step of measuring the amount of the ligand which is bound to the cell membrane of the cells and comparing this amount of the ligand with the amount of the ligand in control cells not brought into contact with the test substance; and
   (c) a step of selecting a substance capable of activating CaSR based on the comparison results of the above-mentioned (b).

The amount of the known ligand can be determined by subjecting a part of those substances to radioactive labeling, and determining the amount of radioactivity bound to the cell membrane.
In the step (c) of the above-mentioned screening method, the comparison of the amounts of ligand is conducted based on, for example, the presence or absence of a significant difference. As a result of the evaluation of the amount of the ligand, if it can be confirmed that the amount of the ligand bound decreases, the test substance can be assessed as a CaSR agonist or modulator.

(3) A method containing the following steps (a), (b), and (c):
   (a) a step of bringing a test substance into contact with CaSR-expressing cells into which a cAMP-sensitive fluorescent protein (for example, FICRhR or the like) has been introduced for a certain period of time;
   (b) a step of measuring the fluorescence intensity (intracellular cAMP concentration) in the cells brought into contact with the test substance and comparing this intensity with the intensity in control cells not brought into contact with the test substance; and
   (c) a step of selecting a substance capable of activating CaSR based on the comparison results of the above-mentioned (b).

In the step (a) of the above-mentioned screening method, in the case of screening a CaSR modulator, the test substance and a CaSR agonist can be brought into contact with the CaSR-expressing cells into which the cAMP-sensitive fluorescent protein (for example, FICRhR or the like) has been introduced.

In the step (b) of the above-mentioned screening method, in the case of screening a CaSR modulator, the fluorescence intensity (intracellular cAMP concentration) in the case of bringing a CaSR agonist into contact with the cells in the presence of the test substance can be compared with the fluorescence intensity (intracellular cAMP concentration) in the case of bringing the CaSR agonist into contact with the cells in the absence of the test substance.

In the step (c) of the above-mentioned screening method, the comparison of the fluorescence intensities is conducted based on, for example, the presence or absence of a significant difference. As a result of the evaluation of the fluorescence intensity, if it can be confirmed that the fluorescence intensity increases, the test substance can be assessed as a CaSR agonist. In the case of screening a CaSR modulator, a substance which extends the increase in the fluorescence intensity can be selected as a CaSR modulator.

The CaSR activator (CaSR agonist or modulator) obtained as above is preferably confirmed to have an immunostimulating activity. The immunostimulating activity can be confirmed, for example, by a method using, as an indicator, an action of promoting the production of IgA or IgG as described in EXAMPLES or cytokines such as IL-6 or IFN-γ, which cytokines have activity to modulate antibody production.

Specific examples of the CaSR agonist include various peptides such as y-Glu-X-Gly (X represents an amino acid or an amino acid derivative other than Cys), γ-Glu-Val-Y (Y represents an amino acid or an amino acid derivative), γ-Glu-Ala, γ-Glu-Gly, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-y-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, and γ-Glu-Cys(S-Me); amino acids such as phenylalanine and tryptophan; cations having a valence of two or more such as calcium and gadolinium; proteins such as protamine; basic peptides such as polylysine; polyamines such as spermine, spermidine or putrescine; various low molecular weight compounds such as cinacalcet and cinacalcet analogous compounds; and the like.

It should be noted that, in the present description, each of the amino acids which constitutes a peptide is in the L-form unless otherwise stated. The term "amino acid" herein includes neutral amino acids such as Gly, Ala, Val, Leu, Ile, Ser, Thr, Cys, Met, Asn, Gln, Pro, or Hyp; acidic amino acids such as Asp or Glu; basic amino acids such as Lys, Arg, or His; aromatic amino acids such as Phe, Tyr, or Trp; as well as homoserine, citrulline, ornithine, α-aminobutyric acid, norvaline, norleucine, taurine, and the like.

In the present description, abbreviations for amino acid residues mean the following amino acids:
- (1): Gly: Glycine
- (2): Ala: Alanine
- (3): Val: Valine
- (4): Leu: Leucine
- (5): Ile:Isoleucine
- (6): Met: Methionine
- (7): Phe: Phenylalanine
- (8): Tyr: Tyrosine
- (9): Trp: Tryptophan
- (10): His: Histidine
- (11): Lys: Lysine
- (12): Arg: Arginine
- (13): Ser: Serine
- (14): Thr: Threonine
- (15): Asp: Aspartic acid
- (16): Glu: Glutamic acid
- (17): Asn: Asparagine
- (18): Gln: Glutamine
- (19): Cys: Cysteine
- (20): Pro: Proline
- (21): Orn: Ornithine
- (22): Sar: Sarcosine
- (23): Cit: Citrulline
- (24): N-Val: Norvaline
- (25): N-Leu: Norleucine
- (26): Abu: α-Aminobutyric acid
- (27): Tau: Taurine
- (28): Hyp: Hydroxyproline
- (29): t-Leu: tert-Leucine

Also, the term, "amino acid derivatives" refers to various derivatives of the above-mentioned amino acids. Examples thereof include an unusual amino acid, a non-natural amino acid, an amino alcohol, or an amino acid in which an amino acid side chain such as the terminal carbonyl group, the terminal amino group, or the thiol group of cysteine is replaced with any one of various substituents. Examples of the substituents include an alkyl group, an acyl group, a hydroxyl group, an amino group, an alkylamino group, a nitro group, a sulfonyl group, various protection groups, and the like. For example, Arg(NO₂): N-γ-nitroarginine, Cys(SNO): S-nitrocysteine, Cys(S-Me): S-methylcysteine, Cys(S-allyl): S-allylcysteine, Val-NH₂: valinamide, Val-ol: valinol (2-amino-3-methyl-1-butanol) and the like are included.

It should be noted that the "(O)" in the above-mentioned γ-Glu-Met(O) and γ-Glu-Cys(S-Me)(O) indicates a sulfoxide structure. The "γ:gamma" in γ-Glu indicates that the glutamic acid binds to another amino acid via the carboxy group at the γ position of the glutamic acid.

In the peptide, "X" is preferably Cys(SNO), Cys(S-allyl), Gly, Cys(S-Me), Abu, or Ser, and "Y" is preferably Gly, Val, Glu, Lys, Phe, Ser, Pro, Arg, Asp, Met, Thr, His, Orn, Asn, Cys, or Gln. However, "X" and "Y" are not limited thereto.

A commercially available product can be used as the above-described peptide. Further, the peptide can be obtained by appropriately employing a known technique such as (1) a chemical synthesis method or (2) a synthesis method through an enzymatic reaction. The peptide to be used in the present invention contains 2 to 3 amino acid residues, i.e., is relatively short, and hence, the chemical synthesis method is convenient. In the case of the chemical synthesis, the oligopeptide can be synthesized or semi-synthesized by using a peptide synthesizer. An example of the chemical synthesis method is a peptide solid phase synthesis method. The peptide synthesized as described above can be purified by general means such as ion exchange chromatography, reversed phase high performance liquid chromatography, or affinity chromatography. Such a peptide solid phase synthesis method and the subsequent peptide purification are well known in the technical field.

Further, the peptide to be used in the present invention can also be produced by an enzymatic reaction. For example, the method described in WO 2004/011653 A1 can be employed. That is, the peptide can also be produced by allowing one amino acid or dipeptide in which the carboxyl terminus of the amino acid or dipeptide is esterified or amidated and an amino acid having a free amino group (for example, an amino acid whose carboxyl group is protected) to react with each other in the presence of a peptide-producing enzyme, and purifying the produced dipeptide or tripeptide. Examples of the peptide-producing enzyme include a culture of a microorganism having an ability to produce the peptide, microbial cells separated from the culture, a processed product of the microbial cells, and a peptide-producing enzyme derived from the microorganism.

Besides such enzymatic method and chemical synthesis method as described above, there are some cases where the peptide to be used in the present invention exists in plants such as vegetables or fruits, microorganisms such as yeast, yeast extract, or the like. In cases where the peptide is a naturally exists, the peptide extracted from these natural products can be used.

In addition, the peptide does not need to be isolated before use, and a fraction containing a large amount of the peptide of the present invention can be used.

Examples of the low molecular weight compound include cinacalcet ((R)-N-(3-(3-(trifluoromethyl)phenyl)propyl)-1-(1-naphthyl)ethylamine) and analogous compounds thereof. Examples of the analogous compounds of cinacalcet include the compound represented by the following chemical formula (1) ((R)-N-[(4-ethoxy-3-methylphenyl)methyl]-1-(1-naphthyl)ethylamine)), the compound represented by the following chemical formula (2) ((R)-N-(3-phenylprop-2-enyl)-1-(3-methoxyphenyl)ethylamine), and the like. These compounds can be synthesized, for example, by such a known method as described in U.S. Patent No. 6211244. In addition, a commercially available product can be used.

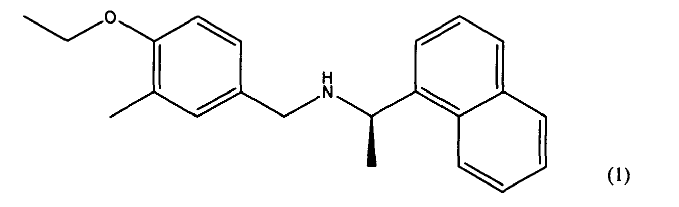

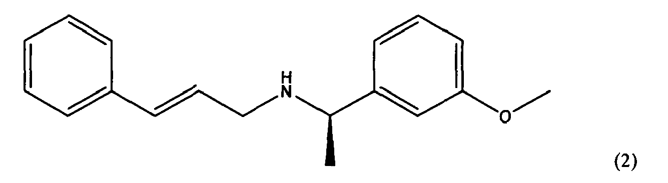

Besides, examples of CaSR activators include a compound described in U.S. Patent No. 6211244, WO06/123725, WO05/115975, U.S. Patent No. 6313146, U.S. Patent No. 6213146, U.S. Patent No. 5688938, U.S. Patent No. 5763569, U.S. Patent No. 5858684, U.S. Patent No. 5962314, U.S. Patent No. 6001884, U.S. Patent No. 6011068, Specification of U.S. Patent No. 6031003, WO95/11221, WO1996/012697, WO2002/059102 or Japanese Patent Application Laid-Open Publication No. 1999-130737.

In the present invention, among the above-mentioned CaSR activators, γ-Glu-Val and cinacalcet are particularly preferred.
A part of or all of the CaSR activator can also be used in the form of a salt as well as a free compound. The CaSR activator as used herein is a concept that encompasses both the free compound and a salt thereof. Examples of the form of the salt include acid addition salts and salts with a base, and it is preferred to select a salt acceptable as a pharmaceutical or a food. Examples of a compound forming the salt acceptable as a pharmaceutical or a food or beverage include an inorganic salt such as a hydrochloride, a hydrobromide, a sulfate, or a phosphate, or an organic salt such as an acetate, a lactate, a citrate, a tartrate, a maleate, a fumarate, or a monomethyl sulfate.

### <2> Immunostimulating Agent

The immunostimulating agent of the present invention contains a CaSR activator as an active ingredient. Examples of the form of the immunostimulating agent of the present invention include pharmaceuticals, quasi-drugs, foods and beverages, and the like.
Examples of an immune system stimulated by the immunostimulating agent of the present invention include mucosal tissues such as the intestinal tract, oral cavity, nose, and respiratory organs, the spleen, and the like. By stimulating these immune systems, immunities are improved. Thus, the immunostimulating agent of the present invention is effective for the treatment or prophylaxis of diseases of which stimulating immunity is effective for the treatment or prophylaxis, such as various infections, diarrhea, polyps, tumors, enteritis, or allergies.

The infections include viral infections and bacterial infections. The viral infections are not particularly limited, and examples thereof include gastrointestinal viral infections (for example, enterovirus and cytomegalovirus), respiratory viral infections (infections caused by respiratory viruses such as influenza virus, rhinovirus, coronavirus, parainfluenza virus, RS virus, adenovirus, and reovirus), herpes zoster caused by herpesvirus, diarrhea caused by rotavirus, viral hepatitis, and AIDS. In the present invention, the immunostimulating agent is particularly effective for the gastrointestinal viral infections.

Further, the bacterial infections are not particularly limited, and examples thereof include infections caused by *Bacillus cereus, Vibrio parahaemolyticus,* enterohemorrhagic *Escherichia coli, Staphylococcus aureus,* MRSA, *Salmonella, Clostridium botulinum,* and

### Candida.

The term "immunostimulation" in the present invention means to activate organism's intrinsic immune system. Specifically, for example, it means to promote the secretion of IgA and/or IgG in immune organs or immune tissues including mucosal tissues of the intestinal tract such as the small intestine; mucosal tissues such as oral cavity, nose, and respiratory organs; and the spleen

Depression of the immunity causes diseases. For example, as symptoms/diseases consistently related to the immunity of the intestinal tracts, infections, allergy diseases, polyps, tumors, enteritis, or the like have been known (Lecture on intestinal immune system, http://www.ioudou.co.jp/col/archives/2004/11/post_7.html).

Also, as shown in EXAMPLES described later, a CaSR activator has an action of promoting the secretion of IgA or IgG. Secretory IgA, for example, specifically binds to microorganisms such as invasive bacteria or viruses; and prevents the adhesion of these microorganisms to epithelial cells. Further, it has been known that secretory IgA has activity to neutralize toxins produced by bacteria such as *Vibrio cholerae* and prevents the absorption of food antigens into a body by binding to allergens contained in foods (Hisako Yasui, Intestinal immune system-regulating action of bifidobacterium,
http://www.healthist.jp/special/150_03/03_03.html).

In addition, it has been known that secretory IgA is involved in the induction of "oral immune tolerance" which is an immunosuppressing mechanism for proteins absorbed from the intestinal tracts (Satoshi Hachimura, Intestinal immune system as contact between food and immune system: Its unique cell responsiveness, http://jsbba.bt.a.u-tokyo.ac.jp/03reikai3/hachimura.pdf) and allergies can be suppressed by inducing the oral immune tolerance.

From these, it is thought that stimulating immunity, for example, promoting the secretion of IgA in the intestinal tracts is effective for the treatment or prophylaxis of the above-mentioned diseases.

Methods for applying the immunostimulating agent of the present invention are not particularly limited. Oral administration, invasive administration using an injection or the like, suppository administration, or transdermal administration can be employed. The active ingredient can be combined with a solid or liquid non-toxic carrier for pharmaceuticals, which carrier is suitable for administration methods such as oral administration, injection, or the like, to administer the active ingredient in the form of a conventional pharmaceutical formulation. Examples of such a formulation include the form of solid drugs such as tablets, granules, powders, or capsules; the form of liquid drugs such as solutions, suspensions, or emulsions; the form of freeze dried drugs; and the like. These formulations can be prepared by a common means in pharmaceutical formulation.

Examples of the above-mentioned non-toxic carrier for pharmaceuticals include glucose, lactose, sucrose, starch, mannitol, dextrin, fatty acid glyceride, polyethylene glycol, hydroxyethylated starch, ethylene glycol, polyoxyethylene sorbitan fatty acid ester, gelatin, albumin, amino acid, water, physiological saline, and the like. Furthermore, if necessary, a commonly used additive such as a stabilizer, a humectant, an emulsifier, a binder, or a tonicity agent can also be appropriately added.

The immunostimulating agent of the present invention can contain, in addition to the CaSR activator, one kind or two or more kinds of other drugs effective for the treatment of a target disease such as a substance having an immunostimulating action or an action of treating or preventing infections. Examples of such a drug include cystine or a derivative thereof, theanine, lactic acid bacteria, and the like.

The dosage or intake of the immunostimulating agent of the present invention can be any amount as long as it is effective for the treatment or prophylaxis and is appropriately adjusted depending on the age, gender, body weight, symptoms, or the like of a patient. For example, in the case of oral administration, it is preferable to be 0.0000001 to 10 g as the amount of the CaSR activator per 1 kg body weight for one administration, and more preferable to be 0.000001 to 1 g per 1 kg body weight. The number of administrations is not particularly limited and the administration can be carried out once to several times per day.

The content of the CaSR activator in the immunostimulating agent of the present invention is not limited as long as it is suitable for the above-mentioned dosage; and is preferably 0.000001 % by mass to 99.9999% by mass per dry weight, more preferably 0.0000 1 % by mass to 99.999% by mass, and particularly preferably 0.000 1 % by mass to 99.99% by mass.

The food or beverage of the present invention contains a CaSR activator. The form of the food or beverage of the present invention is not particularly limited and examples thereof include seasonings, fermented foods, alcoholic beverages, soups, sauces, mayonnaise, dressings, curry roux, juices, nutritional drinks, rice gruel, bread, confectionaries, retort pouch foods, frozen foods, supplements, oral cosmetics, and the like.

The food or beverage of the present invention can be manufactured by the same method using the same raw materials as for manufacturing a general food or beverage except that the CaSR activator is contained. Such raw materials are not particularly limited and examples thereof include rice, barley, corn starch, or the like for alcohol beverages; wheat flour, sugar, salt, butter, yeast for fermentation, or the like for bread; and soy beans, wheat, or the like for fermented foods.

The food or beverage of the present invention preferably contains the CaSR activator in 0.000000001 % by mass or more, more preferably 0.000001 % by mass or more, and particularly preferably 1% by mass or more.

The food or beverage of the present invention has an immunostimulating effect, and for example, the container or package thereof can state that the food or beverage has such an immunostimulating effect or that the food or beverage has a therapeutic effector a prophylactic effect against the above-mentioned diseases.

### EXAMPLES

The invention will now be described more specifically by way of examples. Yet, the present invention is not limited thereto. Please be noted that cinacalcet was synthesized by the method described in the following Production Example 1.

### [Production Example 1]

Synthesis of (R)-N-(3-(3-trifluoromethylphenyl)propyl)-1-(1-naphthyl)ethylamine hydrochloride (Cinacalcet hydrochloride)

### (Step 1) Synthesis of 3-(3-trifluoromethylphenyl)-propionic acid methyl ester

A mixture of 2.20 g of 3-(trifluoromethyl)cinnamic acid, 166 mg of palladium/carbon (10%, wet), and 40 ml of ethanol was stirred overnight under a hydrogen atmosphere at 1 atm. The palladium/carbon was removed by filtration, followed by concentrating the filtrate under reduced pressure. 20 ml of methanol and 4 drops of concentrated sulfuric acid were added and stirred at 60°C for 2 hours, and then allowed to cool down. After concentration under reduced pressure, 20 ml of saturated sodium bicarbonate aqueous solution was added and the resultant was extracted with 20 ml of dichloromethane. The extract was dried with anhydrous sodium sulfate and concentrated under reduced pressure, thereby obtaining 2.40 g of the captioned compound, which was oily.

¹H-NMR (300 MHz, CDCl₃) δ 2.66 (2H, t, J=7.5 Hz), 3.02 (2H, t, J=7.5 Hz), 3.68 (3H, s), 7.37-7.50 (4H, m)

### (Step 2) Synthesis of 3-(3-trifluoromethylphenyl)propanal

2.40 g of 3-(3-trifluoromethylphenyl)-propionic acid methyl ester synthesized in the step 1 was dissolved in 20 ml of dry dichloromethane. Under argon atmosphere, 13 ml of hexane solution of diisopropyl aluminum hydride (0.91 M) was added thereto dropwise at - 78°C over 5 minutes and stirred at the same temperature for 40 minutes. After 50 ml of saturated aqueous ammonium chloride solution was added dropwise, the mixture was stirred and allowed to warm to room temperature. And then 20 ml of water and 5 ml of concentrated hydrochloric acid were added to separate the resultant into layers. The aqueous layer was extracted with dichloromethane and the extract was combined with the separated organic layer. The resultant was dried with anhydrous sodium sulfate and then concentrated under reduced pressure, thereby obtaining 2.12 g of the captioned compound, which was oily.

¹H-NMR (300 MHz, CDCl₃) δ 2.83 (2H, t-t, J=7.5 Hz, 1.2 Hz), 3.02 (2H, t, J=7.5 Hz), 7.36-7.52 (4H, m), 9.83 (1H, t, J=1.2 Hz)

### (Step 3) Synthesis of (R)-N-(3-(3-trifluoromethylphenyl)propyl)-1-(1-naphthyl) ethylamine hydrochloride (Cinacalcet hydrochloride)

To a mixture of 2.12 g of 3-(3-trifluoromethylphenyl)propanal synthesized in the step 2, 2.0 ml of (R)-1-(1-naphthyl)ethylamine, 3.42 g of sodium triacetoxyborohydride, and 150 ml of dry dichloromethane, 0.750 ml of glacial acetic acid was added and the mixture was stirred at room temperature for 5 hours. After 100 ml of water was added thereto and the mixture was stirred for 3 hours, 100 ml of 2 M aqueous sodium hydroxide solution was added to separate the resultant into layers. The aqueous layer was extracted with dichloromethane and the extract was combined with the separated organic layer. The resultant was dried with anhydrous sodium sulfate and then concentrated under reduced pressure. The residue was purified by column chromatography (silica gel/hexane:ethyl acetate of 4:1 to 1:1) and concentrated, thereby obtaining 3.41 g of oily (R)-N-(3-(3-trifluoromethylphenyl)propyl)-1-(1-naphthyl) ethylamine. This was dissolved in 10 ml of dichloromethane. 5 ml of 4 M hydrochloric acid/dioxane and 20 ml of toluene were added thereto and the mixture was subjected to concentration to dryness under reduced pressure. This was recrystallized by 40 ml of ethanol and 200 ml of heptane, thereby obtaining 1.71 g of the captioned compound.

¹H-NMR (300 MHz, DMSO-d⁶) δ 1.69(3H, d, J=6.6 Hz), 2.00 (2H, quintet, J=7.8 Hz), 2.72 (2H, t, J=7.5 Hz), 2.65-2.85 (1H, br), 2.90-3.05 (1H, br), 5.24-5.38 (1H, br), 7.44-7.67 (7H, m), 7.96-8.04 (3H, m), 8.23-8.28 (1H, pseud d), 9.20-9.40 (1H, br), 9.80-10.00 (1H, br) MS (ESI, m/z) 358(MH+)

### [Example 1]

BALB mice (female) of 6 to 8 weeks old were purchased and fed for 1 week for habituation. A commercially available solid feed was used as a feed and tap water was used as water. In order to evaluate influences of cinacalcet and γ-glutamylvaline on antibody producing activity, a culture system using mouse spleen cells was employed. The spleen was collected from BALB-c mice (n=3). The collected cells were washed and thereafter suspended in an RPMI1640 medium (10% FCS, 50 U/ml penicillin, and 50 µg/ml streptomycin). To cells adjusted to 1 × 10⁶ per a 48 well culture plate, cinacalcet or γ-glutamylvaline (dissolved in a sterilized phosphate buffer), or a phosphate buffer as a control was added. And the cells were then stimulated with 5 µg of LPS (lipopolysaccharide, obtained from Sigma). Thereafter, the cells were cultured in a CO₂ incubator at 37°C for 5 days and the amount of IgA antibody in the supernatant was analyzed.

In the same manner, the cells adjusted to 1 × 10⁶ per a 48 well culture plate were, in the presence or absence of cinacalcet or γ-glutamylvaline, stimulated with 0.5 µg of ConA (concanavalin A). Thereafter, the cells were cultured in a CO₂ incubator at 37°C for 5 days and the amount of IgG antibody in the supernatant was analyzed.

The amounts of IgA and IgG were measured by the ELISA method. The method for measuring IgG is shown below. 100 µl of rat anti-mouse IgG antibody (Calbiochem, 1 µg/ml in 50 mM sodium carbonate buffer, pH 8.5) solution was added to a 96 well ELISA plate and the plate was incubated at 4°C overnight to coat each well with the anti-mouse IgG antibody. The plate was washed three times with PBST (phosphate buffered saline, 0.05% Tween 20) and subjected to a blocking treatment with 200 µl of PBS solution containing 2% BSA at 37°C for 1 hr.

The plate was washed three times with PBST. Thereafter, 100 µl of the culture supernatant (9 fold diluted with PBST containing 1% BSA) was added and allowed to react at 37°C for 2 hr. The plate was again washed four times with PBST. 100 µl of an alkaline phosphatase-conjugated anti-mouse IgG (rabbit) (BD Biosciences) solution diluted 2000 fold with PBST containing 1% BSA was added and allowed to react at 37°C for 1 hour. The plate was washed six times and then colored with p-nitrophenyl phosphate. The reaction was terminated with 100 µl/well of 3 M NaOH and the absorbance at 405 nm was measured.

The measurement of IgA was carried out in accordance with the method for IgG except that a rat anti-mouse IgA antibody (BD Biosciences) was used as the immobilized antibody, a biotinylated anti-mouse IgA antibody (rat) (BD Biosciences) was used as the labeled antibody, 3,3',5,5'-tetramethylbenzidine solution (TMB substrate-developing solution, Sigma) was used as the chromogenic substrate, termination of the coloring was carried out using 1 M sulfuric acid, and the absorbance at 450 nm was measured.
A statistical analysis was carried out by Student's t test and a significance level of 5% or less was considered as significant difference.

The results are shown in Table 1 and Table 2. Abilities of cinacalcet and γ-glutamylvaline to promote IgA production upon LPS stimulation were significantly confirmed. Also, abilities to promote IgG production upon ConA stimulation were also significantly confirmed.

**Table 1 Effects on the amounts of IgA production upon LPS stimulation of mouse spleen cells (Alteration in IgA concentration (ng/ml))**

| | Control | LPS | LPS + γ-GluVal (0.1 mM) | LPS + Cinacalcet (0.1 µM) |
|---|---|---|---|---|
| Mean | 4.17 | 16.49 | 25.28 | 26.09 |
| Standard deviation | 0.95 | 2.85 | 2.92 | 4.73 |

**Table 2 Effects on the amounts of IgG production upon ConA stimulation of mouse spleen cells (Alteration in IgG concentration (ng/ml))**

| | Control | ConA | ConA + γ-GluVal (0.1 mM) | ConA + Cinacalcet (0.1 µM) |
|---|---|---|---|---|
| Mean | 7.93 | 41.87 | 62.78 | 55.24 |
| Standard deviation | 0.08 | 6.75 | 6.23 | 3.58 |

### Industrial Applicability

The immunostimulating agent of the present invention is able to safely and effectively stimulate immunity. Also, the food or beverage of the present invention has an immunostimulating action.

## Claims

1. An immunostimulating agent containing a calcium receptor activator.

2. The immunostimulating agent according to claim 1, wherein said calcium receptor activator is selected from the group consisting of γ-Glu-X-Gly (X represents an amino acid or an amino acid derivative other than Cys), γ-Glu-Val-Y (Y represents an amino acid or an amino acid derivative), γ-Glu-Ala, γ-Glu-Gly, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-y-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, γ-Glu-Cys(S-Me), a cation having a valence of two or more, protamine, polylysine, spermine, spermidine, putrescine, cinacalcet, cinacalcet analogous compounds, and salts thereof.

3. The immunostimulating agent according to claim 2, wherein said X is Cys (SNO), Cys(S-allyl), Gly, Cys(S-Me), Abu, or Ser; and said Y is Gly, Val, Glu, Lys, Phe, Ser, Pro, Arg, Asp, Met, Thr, His, Orn, Asn, Cys, or Gln.

4. The immunostimulating agent according to claim 2 or 3, wherein said calcium receptor activator is γ-Glu-Val or cinacalcet.

5. The immunostimulating agent according to any one of claims 1 to 4, which is used as a pharmaceutical for the treatment or prophylaxis of an infection, diarrhea, polyp, tumor, enteritis, or allergy.

6. A food or beverage for immunostimulation containing γ-Glu-Val or cinacalcet in an amount of 0.00000000 1 % by mass or more.
